(19) Europäisches Patentamt
European Patent Office
Office européen des brevets

(11) EP 1 041 074 A1

(12) # EUROPEAN PATENT APPLICATION

(43) Date of publication:
**04.10.2000 Bulletin 2000/40**

(51) Int Cl.[7]: **C07D 491/16**, C09K 11/06

(21) Application number: **00301950.2**

(22) Date of filing: **09.03.2000**

(84) Designated Contracting States:
**AT BE CH CY DE DK ES FI FR GB GR IE IT LI LU MC NL PT SE**
Designated Extension States:
**AL LT LV MK RO SI**

(30) Priority: **09.03.1999 JP 6192199**
**30.06.1999 JP 18517299**
**19.01.2000 JP 2000010904**

(71) Applicant: **KABUSHIKI KAISHA HAYASHIBARA SEIBUTSU KAGAKU KENKYUJO**
**Okayama-shi Okayama (JP)**

(72) Inventors:
- **Taniguchi, Yoshio**
  **Ueda-shi, Nagano (JP)**
- **Koyama, Toshiki**
  **Ueda-shi, Nagano (JP)**
- **Adachi, Chihaya**
  **East Windsor, NJ 08520 (US)**
- **Saitou, Tomoyasu**
  **Urawa-shi, Saitama (JP)**
- **Satsuki, Makoto**
  **Tsukubo-gun, Okayama (JP)**
- **Shinpo, Akira**
  **Oku-gun, Okayama (JP)**
- **Suga, Sadaharu**
  **Tamano-shi, Okayama (JP)**

(74) Representative: **Jenkins, Peter David et al**
**PAGE WHITE & FARRER**
**54 Doughty Street**
**London WC1N 2LS (GB)**

Remarks:
A request for correction of the description has been filed pursuant to Rule 88 EPC. A decision on the request will be taken during the proceedings before the Examining Division (Guidelines for Examination in the EPO, A-V, 3.).

(54) **Light-sensitive 4-cyanocoumarin derivatives**

(57) 4-Cyanocoumarin derivatives which have a distinct sensitivity to visible lights, distinct luminescent ability, and satisfactory applicability for photochemical polymerization, dye lasers, and organic electroluminescence. Since most of the derivatives have a fluorescent maximum at a wavelength of 600 to 650 nm, and particularly at a wavelength of 610 to 630 nm, they are extremely useful as luminescent agents for organic EL elements that emit visible lights in a red-color region.

EP 1 041 074 A1

## Description

## Background of the Invention

### Field of the Invention

The present invention relates to a functional organic compound, and more particularly, to a novel 4-cyanocoumarin derivative useful in a photochemical polymerization, dye laser, and electroluminescence.

### Description of the Prior Art

After coming into this information age, photochemical polymerization has been widely used in many fields beyond the field of synthetic resins, and now it is being extensively used in the fields of information recordings including memories (hereinafter referred to as "recordings") and electronic equipments such as paints, lithographic plates for printing, printing circuits, and integrated circuits. Photochemical polymerization is a technique for polymerizing polymeric compounds by irradiating lights and is roughly classified into (a) photopolymerization that initiates the polymerization of polymeric compounds by directly irradiating them for activation, and (b) photosensitization polymerization that polymerizes polymeric compounds by irradiating them in the presence of photosensitizers to make them into their growth-active forms. In both cases, as characteristic features, the initiation and the suspension of polymerization can be controlled by flashing excited lights, and the rate and the degree of polymerization can be easily controlled by appropriately selecting the strength and the wavelength of the lights. The photochemical polymerization can be proceeded even at a relatively-low temperature because it requires a lesser energy for initiating polymerization.

As a new development of information recordings such as holography and lithographic plates for printing and due to the advantageous features of photochemical polymerization, photopolymeric compositions, which can be polymerized by irradiating visible lights such as those of argon ion lasers, helium/neon lasers, and second harmonics of YAG lasers, are now rapidly in great demand. However, polymeric compounds and polymerization initiators, which are generally incorporated into photopolymeric compositions, absorb ultraviolet rays only, and this inevitably needs photosensitizers as a technical factor when polymerizing the compositions with visible lights. In photopolymeric compositions used for information recordings and electronic equipments, polymeric compounds should be used in combination with photosensitizers, polymerization initiators, and binding resins, which can be selected from materials to suite their use. In general, there may be employed a technique for selecting materials other than photosensitizers and then selecting photosensitizers capable of sensitizing the selected polymeric compounds and/or polymerization initiators in such a manner of trial and error.

Desired features needed for photosensitizers are as follows: They should have a relatively-high molecular absorption coefficient in a visible region, sensitize polymeric compounds and polymerization initiators, exert a relatively-high sensitization efficiency, have a satisfactory solubility in both solvents and another compounds to be incorporated, and have a satisfactory stability. Representative examples of such photosensitizers are merocyanine dyes as disclosed in Japanese Patent Kokai No. 151,024/79, cyanine dyes as disclosed in Japanese Patent Kokai No. 29,803/83, stilbene derivatives as disclosed in Japanese Patent Kokai No. 56,403/84, coumarin derivatives as disclosed in Japanese Patent Kokai No. 23,901/88, pyran derivatives as disclosed in Japanese Patent Kokai No. 329,654/94, and methylene blue derivatives as disclosed in Japanese Patent Kokai No. 33,104/89. All of these compounds have both merits and demerits, and there found no photopolymeric compound, comprising various materials, which can constantly exert the aforesaid characteristic features.

Organic compounds which are sensitive to lights, and more particularly, those which are capable of emitting lights are also useful in the field of dye lasers and electroluminescence.

In the field of dye lasers, as disclosed, for example, by Kaoru IGARASHI in *"Shikizai-Kyokai-shi",* Vol. 70, No. 2, pp. 102-111 (1997), since a report of dye laser's lasing in 1960, compounds which emit lights in a visible region have been eagerly screened. As the progress of information recording technology, compounds which emit lights in a longer wavelength region, and more particularly, those in a visible region, have been needed more and more.

In the field of information displays, electroluminescent elements (hereinafter abbreviated as "EL element") are now highlighted as a display element for the forthcoming generation. At present, cathode-ray tubes are predominantly used in a larger size of information displays such as computer termini and TV receivers. The cathode-ray tubes, however, are relatively-large in mass and weight and relatively-high in operation voltage, and this hinders their applicability for commonly used equipments and smaller size of portable ones where transportability is highly valued. More required are the information displays which are relatively small in size like in a thinner and lighter plain-form and which operate at a lower operation voltage and wattage. Due to advantageous features of relatively-low operation voltage and wattage, liquid crystal elements are now commonly used in many fields. However, the information displaying equipments using such elements will change in contrast when viewed from different angles, display information clearly only

within a specific angle, and require blacklight, resulting in a problem of that they could not be reduced their wattage as high as they are expected. As a display element to overcome these drawbacks, there appeared an electroluminescent element, i.e., an organic EL element.

Organic EL element is generally a luminous element which has a thin film, containing a luminescent agent and being inserted between a cathode and anode, and which utilizes luminescence such as fluorescence or phosphorescence emitted by the luminescent agent in such a manner that a dc voltage is energized between the cathode and anode to supply positive holes and electrons to the film and to couple the positive holes and electrons together to make the agent into an excited state, and the excited agent returns to the ground state while emitting luminescence. As characteristic features, organic EL element can be appropriately changed its luminous color tint by selecting an appropriate host luminous agent and varying a guest luminescent agent used with the host. Depending on the combination of luminous agents as the host and guest, the brightness and the life expectancy of luminescence can possibly be improved by a large margin. It is said that organic EL element is a theoretically excellent luminous-element because of the following merits: Information displaying apparatuses with such organic EL element are free of view angles because the element autonomously emits light, and are low in energy consumption because the element needs no blacklight.

In respect of organic EL elements which emit lights in a green color region, there is a report for improving luminous efficiency by using a luminescent agent as a guest. However, there found no effective luminescent agent usable as a guest in organic EL element for a red color region. Therefore, the latter is far from emitting a complete red luminescence and has a relatively-short life expectancy and poor durability and reliability. For example, organic EL elements, as disclosed in Japanese Patent Kokai No. 6,042/98 and United States Patent No. 4,769,292, can only emit an insufficient brightness and an incomplete red color emission. Accordingly, conventional organic EL elements would still be problematic in realizing a full color emission.

To supply organic EL elements at a relatively-lower cost, it is essential to explore a luminescent agent which does not intrinsically require doping of a guest luminescent agent in addition to simplify the total structure of the elements and to facilitate the vapor deposition in their processings. Many proposals have been made on luminescent agents used for organic EL elements, but there found no compound which fulfills the aforesaid requirements.

## Summary of the Invention

In view of the foregoing, an aim of the present invention is to provide a novel organic compound which has a distinct sensitivity to a visible light and a relatively-high luminescent ability, and its uses in photochemical polymerization, organic EL elements, and dye lasers.

Accordingly, the present inventors eagerly screened compounds and found that a series of 4-cyanocoumarin derivatives, which are obtainable by cyanizing coumarin compounds having a julolidine structure intramolecularly, are highly susceptible to visible lights and extremely useful in photochemical polymerization. They also found that the 4-cyanocoumarin derivatives exert a distinct luminescent ability in a visible region and can be advantageously used in dye lasers and organic EL elements. The present invention was made based on the preparation of the novel 4-cyanocoumarin derivatives and the finding of their industrially useful properties.

The present invention achieves the above aim by providing the novel 4-cyanocoumarin derivatives.

## Detailed Description of the Invention

The present invention relates to the 4-cyanocoumarin derivatives represented by Formula 1:

Formula 1

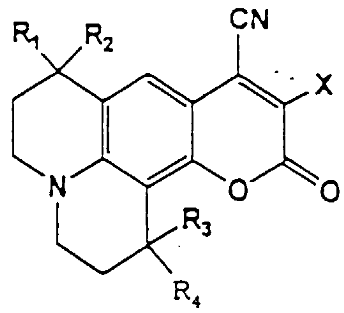

In Formula 1, $R_1$ to $R_4$ independently denote a hydrogen atom, aliphatic hydrocarbon group or aromatic hydrocarbon group, wherein the hydrocarbon groups may contain a substituent. Depending on use, when $R_1$ to $R_4$ are aliphatic hydrocarbon groups, the chain length is generally selected from those having carbon atoms of up to five, and preferably those having 1 to 4 carbon atoms. The aliphatic hydrocarbon groups may be those in a linear or branching form; examples of such are a methyl, ethyl, vinyl, propyl, isopropyl, 1-propenyl, 2-propenyl, isopropenyl, butyl, isobutyl, sec-butyl, tert-butyl, 2-butenyl, 1,3-butadienyl, pentyl, isopentyl, neopentyl, and 2-pentenyl groups. Examples of the aromatic hydrocarbon group used in the present invention are a phenyl, tolyl, xylyl, biphenyl, naphthyl, anthryl, and phenanthryl groups, which may have one or more of substituents. Examples of these substituents are a carboxyl, alkylcarbonyloxy, alkoxy, alkoxycarbonyl, sulfonyl, alkylsulfonyl, aminosulfonyl, hydroxyl, aromatic hydrocarbon, alicyclic hydrocarbon, cyano, 2-butoxyethyl, 2-(2-ethoxy)ethoxyethyl, 2-cyanoethyl, 6-bromohexyl, 2-carboxyethyl, 3-sulfoxypropyl, 4-sulfoxybutyl, 2-hydroxyethyl, phenylmethyl, 4-butoxyphenylmethyl, and 4-butylphenylmethyl groups.

In Formula 1, X denotes a heterocyclic group such as a monocyclic heterocyclic group including a five-ring group which comprises one or more nitrogen, oxygen, sulfur and/or selenium atoms within the ring; or a polycyclic heterocyclic group formed by condensation of an aromatic hydrocarbon group such as a benzene, naphthalene, anthracene, phenanthrene, or pyrene ring. Examples of the heterocyclic groups are monocyclic groups such as a thiazolyl, oxazolyl, imidazolyl group, and triazolyl groups; and polycyclic heterocyclic groups such as a benzothiazolyl, benzoselenazolyl, benzoxazolyl, benzoimidazolyl, naphtothiazolyl, naphtoselenazolyl, naphtoxazolyl, napthoimidazolyl, phenathrothiazolyl, and pyrenothiazolyl groups. These heterocyclic groups may contain one or more substituents of halogen groups such as a cyano, fluoro, chloro, bromo, and iodo groups; alkoxy groups such as a methoxy and ethoxy groups; aromatic hydrocarbon groups such as an alkoxy, phenyl, bromophenyl, tolyl, xylyl, biphenyl, paphtyl, anthryl, and phenanthryl groups; halogen-substituted aromatic hydrocarbon groups; aromatic hydrocarbon groups such as a methyl, trifluoromethyl, ethyl, propyl, isopropyl, and butyl groups having carbon atoms up to four; and halogen-substituted aliphatic hydrocarbons.

The 4-cyanocoumarin derivatives of the present invention should not be restricted to those having only one 4-cyanocoumarin structure within a molecule as represented by Formula 1, and they may include those having two or more 4-cyanocoumarin structures per one molecule as long as they satisfy the object of the present invention. Of course, these plural structures may be identical or different, or may be symmetric or non-symmetric as the whole molecules. A plurality of 4-cyanocoumarin structures in the same molecule can be linked each other, for example, via characteristic groups containing hetero-atoms such as an oxygen, sulfur, and nitrogen atoms, for example, an oxy, thio, imino, and azo groups; or via appropriate-length of aliphatic hydrocarbon groups which may have the above characteristic groups within the chains; or linked via the condensation of heterocyclic groups as represented by Formula 1. These 4-cyanocoumarin derivatives have characteristics of a significantly-high ability of emitting light per molecule and a significantly-high sensitivity to visible lights as compared with the compound represented by Formula 1 having a single 4-cyanocoumarin structure within the molecule.

Preferred examples of the 4-cyanocoumarin derivatives of the present invention are the compounds represented by Formulae 3 to 33. For the use in photopolymeric compositions, dye lasers, or organic EL elements, the 4-cyanocoumarin derivatives represented by Formula 1 where $R_1$ to $R_4$ are identical or differently aliphatic C 1-4 hydrocarbon groups, and where X is a polycyclic heterocyclic group having a heterocyclic five-ring within the ring. The compounds, which have the above features and a clear sensitivity to visible lights, and emit light in a visible region, such as those represented by Formulae 3, 9, 10, 11, 12 and 28 are extremely useful as photosensitizers for the photochemical condensation of polymeric compounds, laser-active substances in dye lasers, and guests luminous agents for doping host luminescent agents in organic EL elements and for another host luminescent agents.

Formula 3

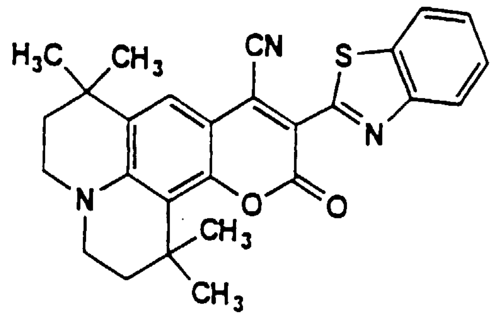

Formula 4

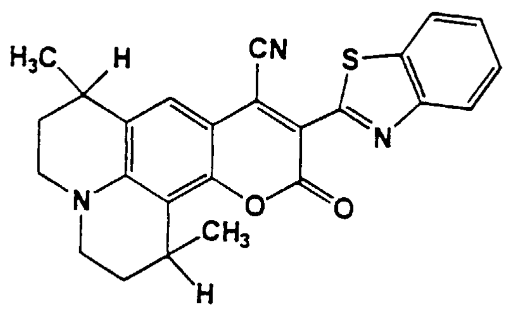

Formula 5

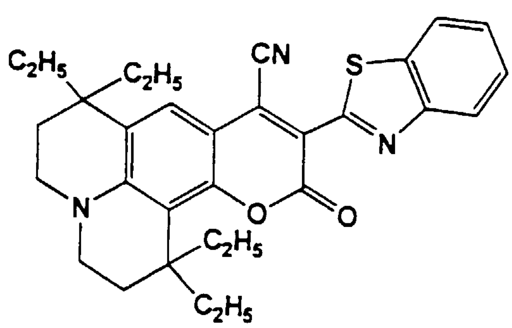

Formula 6

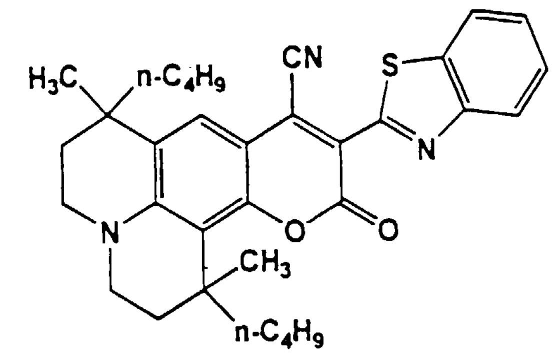

Formula 7

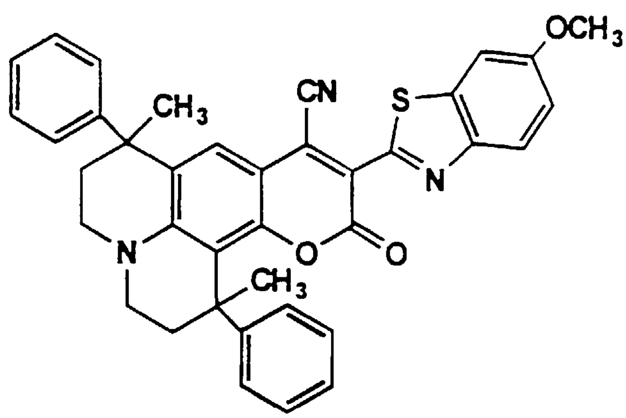

Formula 8

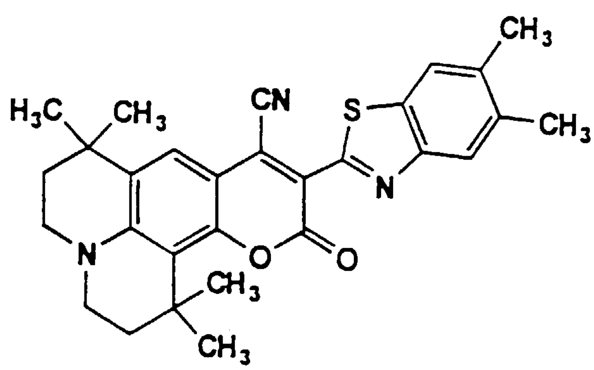

Formula 9

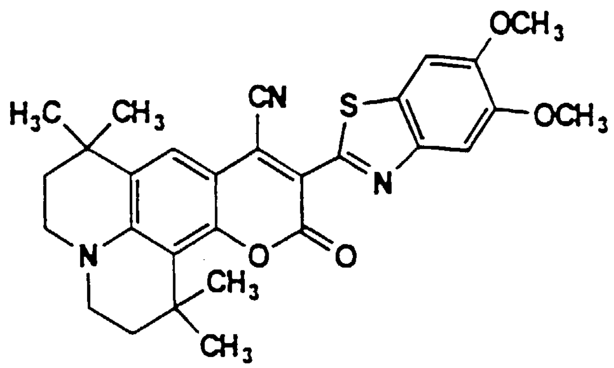

Formula 10

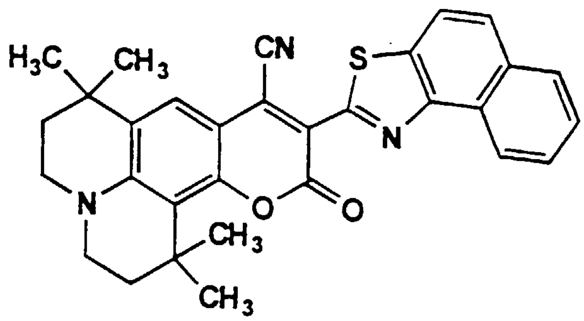

Formula 11

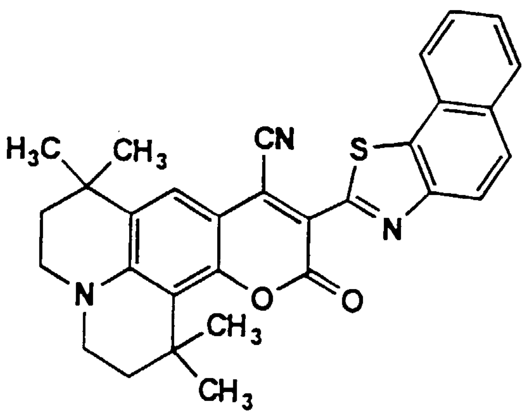

Formula 12

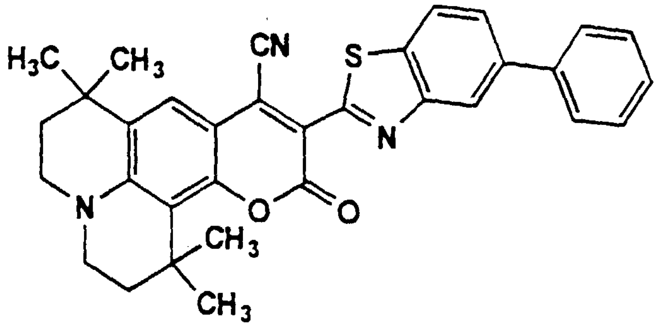

Formula 13

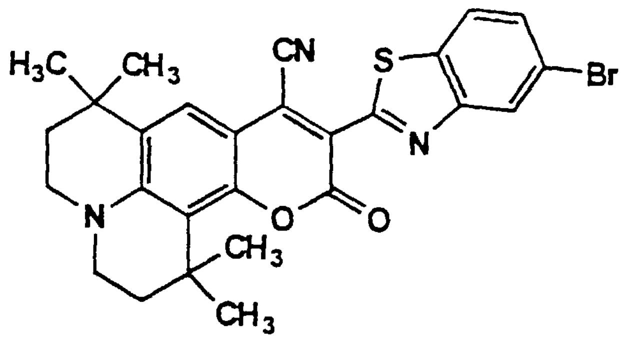

Formula 14

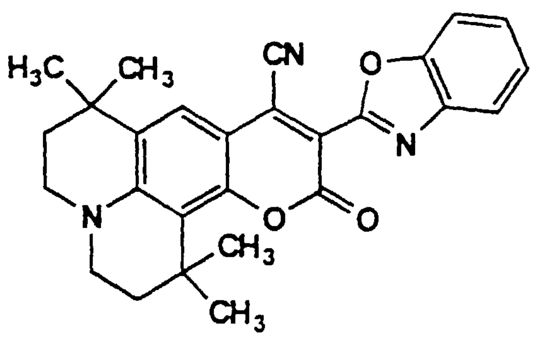

Formula 15

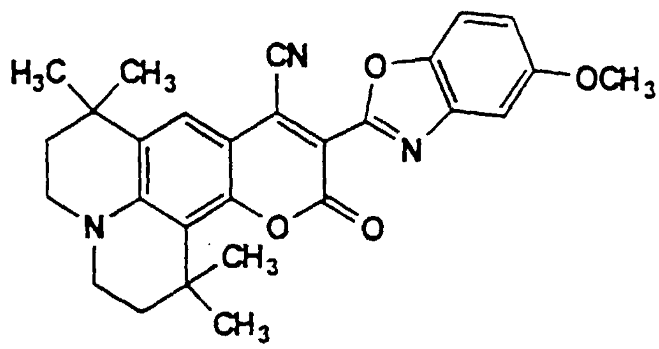

Formula 16

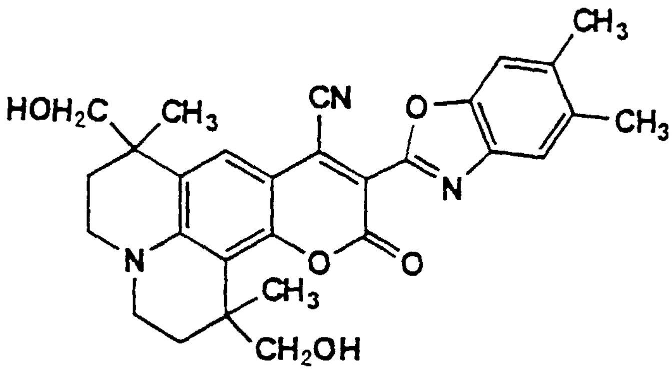

Formula 17

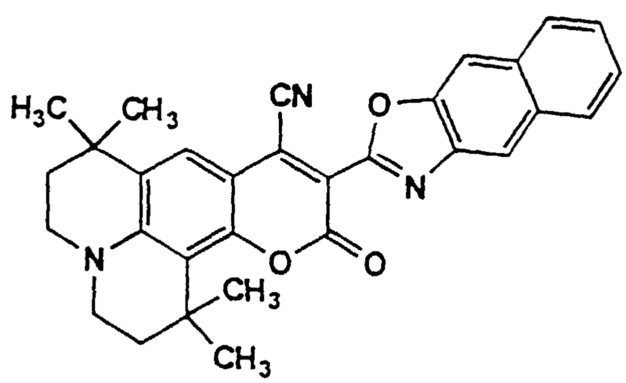

Formula 18

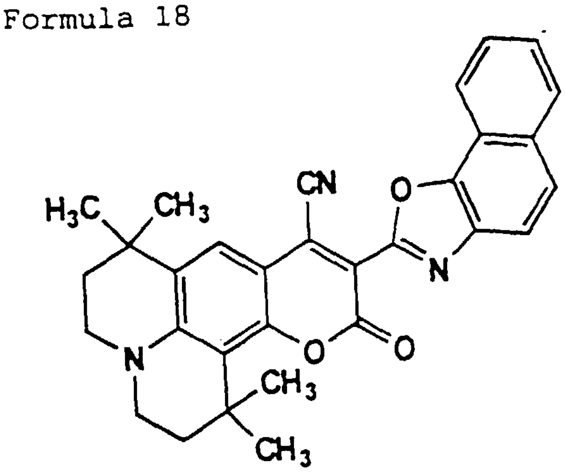

Formula 19

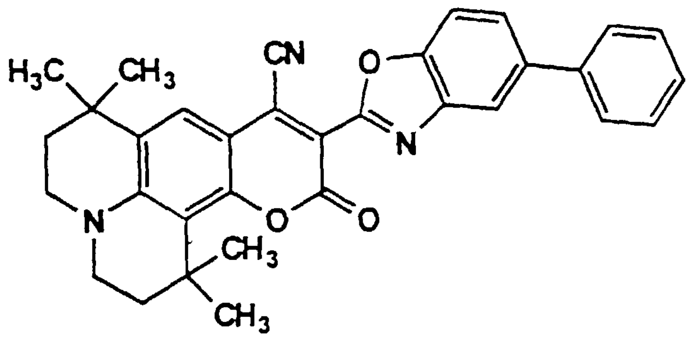

Formula 20

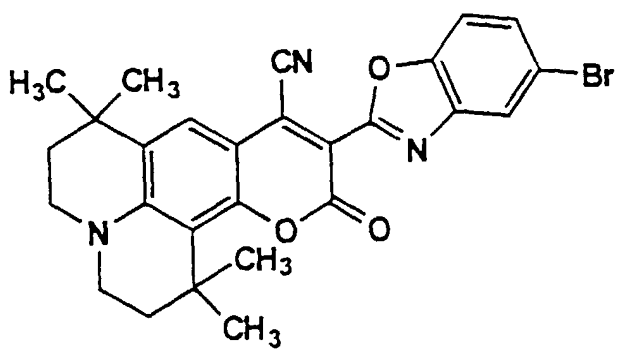

Formula 21

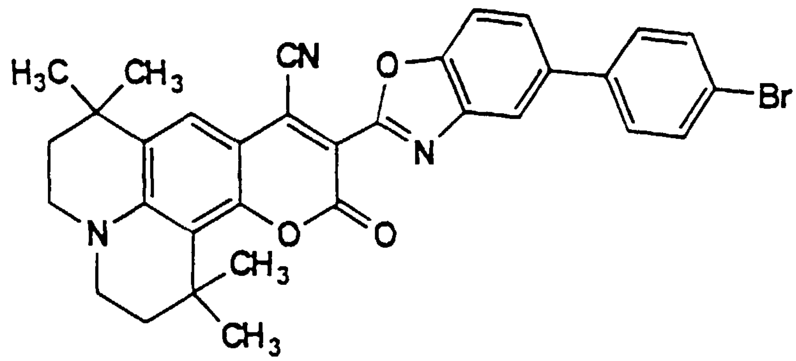

Formula 22

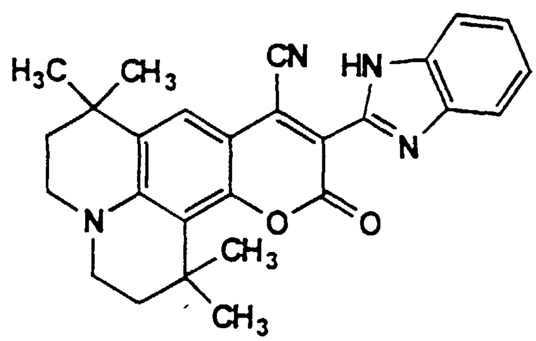

Formula 23

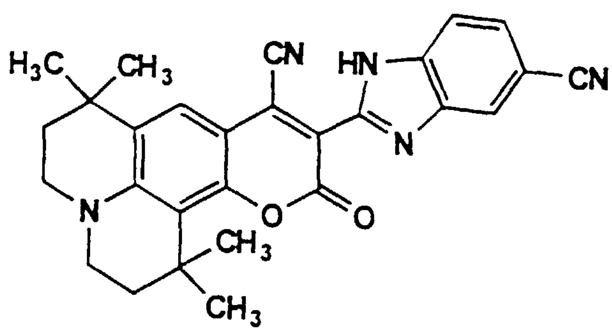

Formula 24

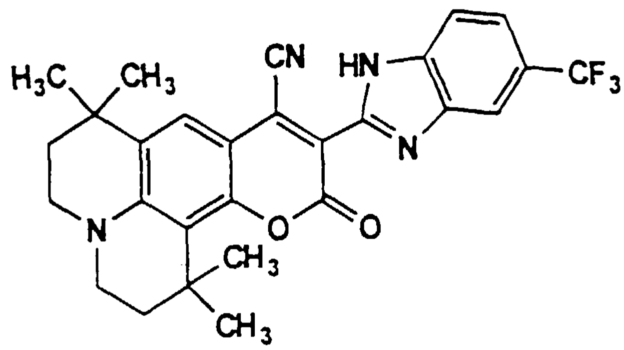

Formula 25

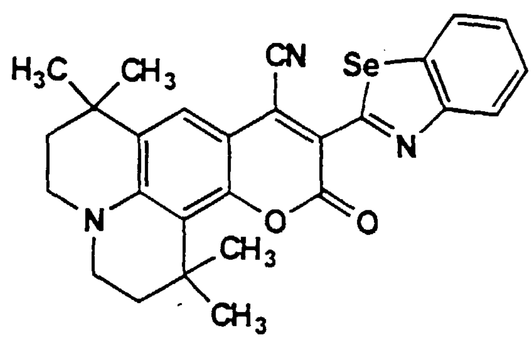

Formula 26

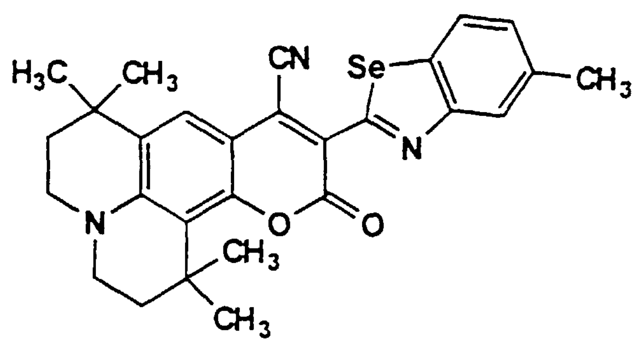

Formula 27

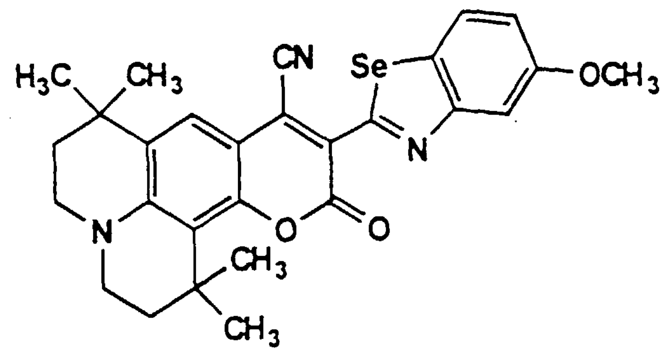

Formula 28

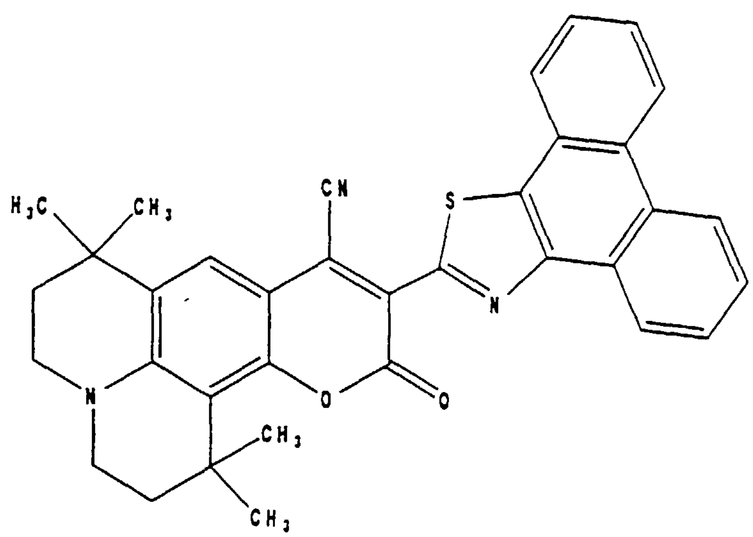

Formula 29

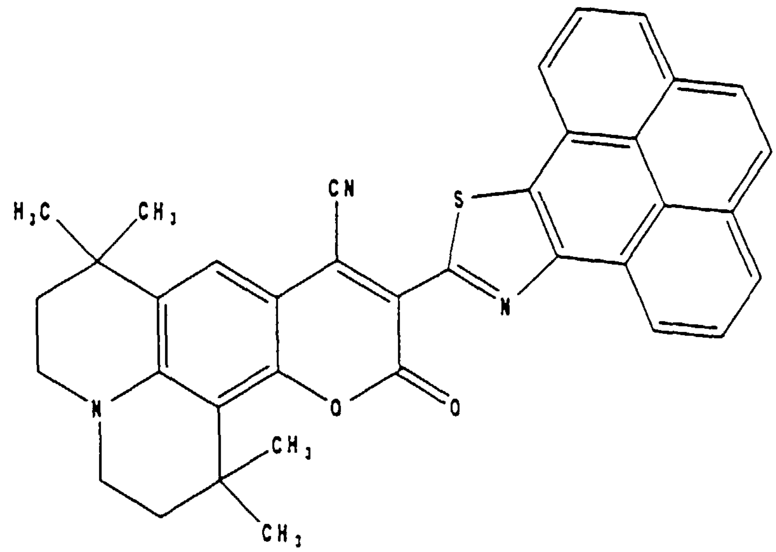

Formula 30

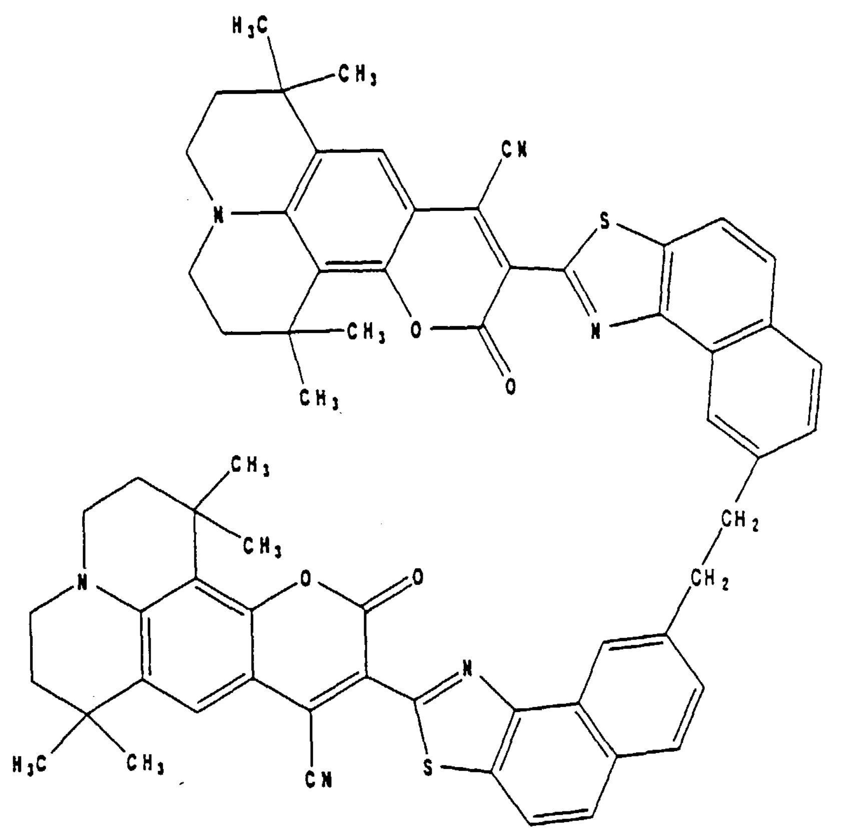

Formula 31

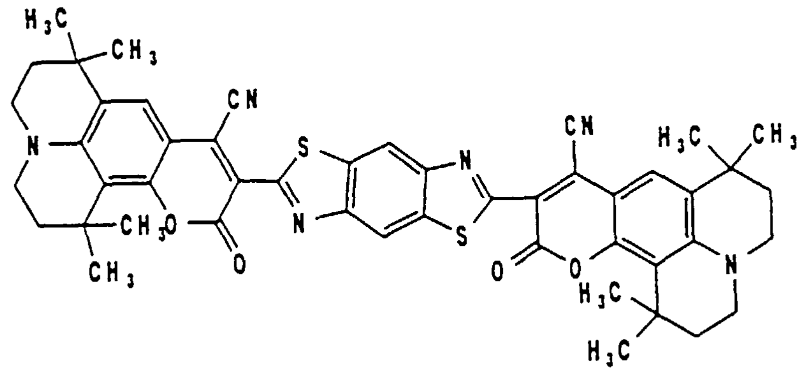

Formula 32

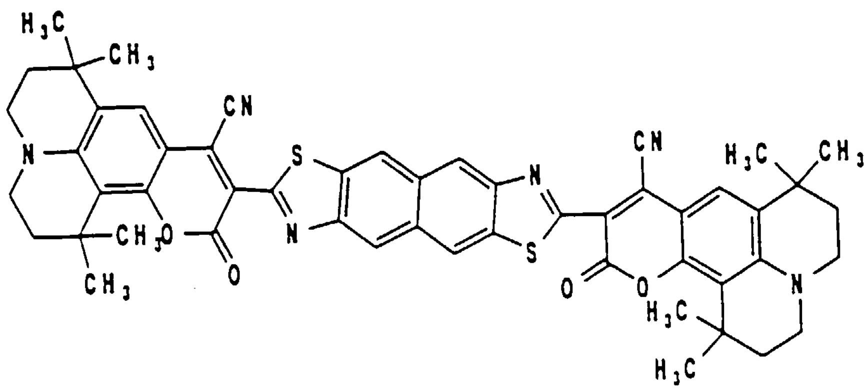

Formula 33

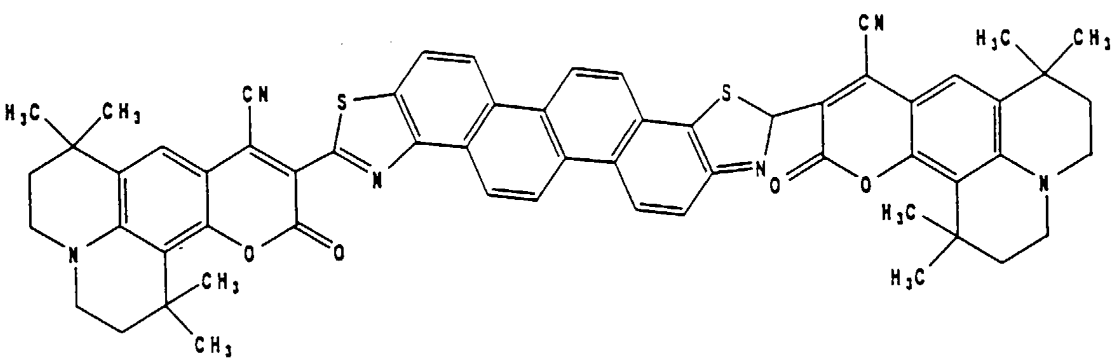

The 4-cyanocoumarin derivatives of the present invention can be prepared by different methods. With an economical viewpoint, preferably used are the methods which produce the derivatives through a step of contacting the compounds, represented by Formula 2 having $R_1$ to $R_4$ which correspond to those in Formula 1, with cyanization agents such as sodium cyanide, potassium cyanide, and cyanohydrin to effect 4-cyanization in a solvent system of a water-soluble solvent(s) such as chloroform, methanol, ethanol, propanol, acetone, ethyl methyl ketone, acetonitrile, propionitrile, tetrahydrofuran, tetrahydropyran, triethylamine, N,N-dimethylformamide, N,N-dimethylacetamide, pyridine, N-methylpyrrolidone, hexamethylphosphoric triamide, sulfolane, and/or dimethylsulfoxide; or in a mixture solutions of

water and the water-soluble solvent(s). Any one of the 4-cyanocoumarin derivatives represented by Formulae 3 to 33 can be prepared by the above methods in a desired amount; the compound represented by Formula 2 can be easily prepared by the method disclosed in Japanese Patent Kokai No. 9,892/94, applied for by the same applicant as the present invention.

Formula 2

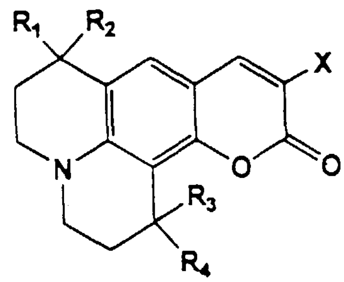

Depending on use, the 4-cyanocoumarin derivatives thus obtained can be used in a reaction mixture form without any further processing, and usually used after purified by the methods generally used in the related compounds thereof such as separation, decantation, filtration, extraction, concentration, thin-layer chromatography, column chromatography, gas chromatography, high-performance liquid chromatography, distillation, sublimation, and crystallization; and if necessary these methods can be used in combination. For use in photosensitizers, the 4-cyanocoumarin derivatives should at least preferably be distilled and/or crystallized prior to use. When used as luminescent agents for organic EL elements or for laser-active substances used in laser oscillation apparatuses, the 4-cyanocoumarin derivatives should preferable be further purified, for example, by sublimation before use.

As described above, the 4-cyanocoumarin derivatives of the present invention are extremely useful as photosensitive agents for polymerizing polymeric compounds with visible lights because they have a distinct sensitivity to a longer wavelength of lights, particularly visible lights, and effectively sensitize polymeric compounds and polymerization initiators. In an actual use, the photosensitizers comprising the 4-cyanocoumarin derivatives of the present invention can be formulated into compositions by combining with materials commonly used in photopolymeric compositions such as polymeric compounds, polymerization initiators, and binding resins. Depending on the types of or the final use of the 4-cyanocoumarin derivatives used, the above polymerization initiators and/or binding resins may be omitted.

The polymeric compounds, for which the photosensitizers of the present invention can be applied, include, for example, monomers, oligomers, prepolymers, and mixtures thereof which contain intermolecularly at least one polymeric-multiple-bond such as an ethylene double bond. Examples of such polymeric compounds are ethyl acrylate, hydroxyethyl acrylate, ethylene glycol dimethacrylate, pentaerythritol triacrylate, pentaerythritol trimethacrylate, polyester methacrylate, polyurethane methacrylate, epoxy methacrylate, etc. The polymeric compounds usable in the photopolymeric compounds of the present invention should not be restricted to the above-mentioned compounds, and should include all the polymeric compounds which can be photopolymerized by the photosensitizers of the present invention.

The polymerization initiators usable in the present invention include organic peroxides such as di-*t*-butyldioxyisophtalate, 3,3',4,4'-tetrakis(*t*-butyldioxycarbonyl)benzophenone, ethyl methyl ketone, 2,5-dimethyl-2,5-bis(*t*-butyldioxy)-3-hexane, di-*t*-butylhydroperoxide, 2,5-bis(hydroperoxy)-2,5-dimethylhexane, *t*-butylhydroperoxide, butyl-4,4-bis(*t*-butyldioxy)valylate, and 1,1-bis(*t*-butyldioxy)-3,3,5-trimethylcyclohexane; hydrocarbon halides such as 2,4,6-trichloromethyl-*s*-triazine; and other compounds widely used in photochemical polymerization such as bisimidazole, benzoyl alkyl ether, iron-allene complex, titanocene compound, N-phenylglycine, and diphenyliodonium salt, which can be used in combination, if necessary.

Depending on use, the binding resins usable in the present invention can be those which are commonly used in photopolymeric compositions; poly-N-vinylpyrrolidone, poly(vinyl acetate), poly(vinyl butyral), poly(vinyl carbazole), polystyrene, poly(methyl methacrylate), poly(ethylene oxide), poly(butyl methacrylate), styrene-maleic esther, poly(methyl methacrylate)methacrylic acid, poly-N-vinylpyrrolidone-glycidyl methacrylate, etc.

The photosensitizers of the present invention comprise one or more specific 4-cyanocoumarin derivatives. To produce photopolymeric compositions by using the photosensitizers, to one part by weight of one of the photosensitizers 1-1,000 parts by weight of, and preferably, 10-500 parts by weight of the aforesaid polymeric compound(s); and optionally up to 1,000 parts by weight of, and preferably up to 500 parts by weight of a biding resin(s), along with 0.1-10 parts by weight of, and preferably 0.5-5 parts by weight of a polymerizing initiator(s). If necessary, the following materials can be appropriately added to the above photopolymeric compositions; quinone or phenol thermal-polymerization-forbidding-agents such as hydroquinone, pyrogallol, 2,6-di-*t*-butyl-*p*-cresol, etc.; plasticizers such as saturated and unsaturated carboxylates such as phthalates and adipates; and coloring agents, preservatives, stabilizers, surface protectants, lubricants, suppository coatings, etc.

The photopolymeric compositions of the present invention can be usually used by dissolving in appropriate solvents into solutions, applying the solutions over appropriate support members, and drying the applications. Examples of such solvents include hydrocarbons such as pentane, hexane, cyclohexane, petroleum ether, petroleum benzine, isooctane, benzene, toluene, and xylene; halogen compounds such as carbon tetrachloride, chloroform, 1,2-dichloroethane, 1,2-bromoethane, trichloroethylene, tetrachloroethylene, chlorobenzene, and α-dichlorobenzene; alcohols and phenols such as methanol, ethanol, 1-propanol, 2-propanol, 2,2,2-trifluoroethanol, 1-butanol, 2-butanol, isobutyl alcohol, isopentyl alcohol, cyclohexanol, ethylene glycol, propylene glycol, 2-methoxyethanol, 2-ethoxyethanol, phenol, benzyl alcohol, cresol, diethylene glycol, triethylene glycol, and glycerine; ethers such as diethyl ether, diisopropyl ether, tetrahydrofuran, tetrahydropyran, 1,4-dioxane, anisole, 1,2-dimethoxyethane, diethylene glycol dimethyl ether, dicyclohexyl-18-crown-6, diethylene glycol monomethyl ether, diethylene glycol monoethyl ether, propylene glycol monomethyl ether, and propylene glycol monomethyl ether acetate; ketones such as acetone, ethyl methyl ketone, and cyclohexanone; esters such as ethyl acetate, butyl acetate, ethyl propionate, ethylene carbonate, propylene carbonate, and triethyl phosphate; amides such as formamide, N-methylformamide, N,N-dimethylformamide, N-methylacetamide, N,N-dimethylacetamide, and hexamethylphosphoric triamide; nitriles such as acetonitrile, propionitrile, succinonitrile, and benzonitrile; nitro compounds such as nitromethane and nitrobenzene; amides such as ethylenediamine, pyridine, piperidine, and morpholine; and sulfur compounds such as dimethylsulfoxide and sulfolane. Depending on use, these solvents can be used in combination.

The support members usable in the present invention can be commercially available ones. Depending on use, the followings can be selectively used; metals such as aluminum, magnesium, copper, zinc, chromium, nickel, and iron, as well as alloying sheets thereof; papers such as high-quality papers, art papers, and parting papers; inorganic sheets made of glasses and/or ceramics; and plastic sheets made of poly(ethylene phthalate), polyethylene, poly(methyl methacrylate), poly(vinyl chloride), vinyl chloride/vinylidene chloride copolymer, poly styrene, nylon, cellulose acetate, cellulose acetate, and/or cellulose acetate butylate.

The polymerization methods used in the present invention should not be restricted to a specific one, any one of the following techniques can be used; photoinitiation polymerization where light relates only to the initiation step for radical polymerization, ion-polymerization, and ring-opening polymerization; and photo-polyaddition polymerization where light relates to the growing step for the polymerization. The lighting source for exposure used in the above techniques may be, for example, sun light, carbon arc, high-pressure mercury lamp, xenon lamp, metal halide lamp, fluorescent lamp, tungsten lamp, argon ion laser, krypton ion laser, helium/cadmium laser, helium neon laser, semiconductor laser, or a commercially available lighting device, which emits visible lights of a wavelength of over 400 nm such as a second harmonic of YAG laser.

The present invention also provides the use of the 4-cyanocoumarin derivatives as luminescent agents for organic EL elements. Since the 4-cyanocoumarin derivatives of the present invention emit lights in a visible region and mostly have a fluorescent maximum at a wavelength of 600 to 650 nm, and particularly at a wavelength of 610 to 630 nm, they are extremely useful as luminescent agents for organic EL elements that emit visible lights in a red-color region. The organic EL elements, for which the 4-cyanocoumarin derivatives can be advantageously applied, are substantially those which comprise organic compounds capable of emitting lights, and most preferably, multi-layer organic EL elements which usually comprise a cathode energized with a positive voltage and an anode energized with a negative voltage, a hole injection/transportation layer for injecting holes from the cathode and transporting the injected holes, an electron injection/transporting layer for injecting electrons from the anode and transporting the injected electrons, and a luminous layer for emitting light by recombining the holes and electrons. The 4-cyanocoumarin derivatives, which distinctively emit lights and form a stable thin-membrane in a glass state, can be advantageously used as host luminescent agents for organic EL elements. Most of the 4-cyanocoumarin derivatives of the present invention function as materials for hole injection/transportation layers and electron injection/transportation layers, and also function as guest luminescent agents for improving luminescent efficiency and luminescent spectra by doping with a small amount to another host luminescent agents such as metal complexes containing 8-quinols as a ligand such as tris-(8-quinolinolato)aluminum. Thus, when used in organic EL elements which inevitably require at least one of these materials, the derivatives of the present invention can be advantageously used alone or in combination with another luminescent agents such as dicyanomethylene, coumarin, perylene, rubrene, and their related compounds; and materials for hole or electron injection/transportation. In some multi-layer organic EL elements where luminescent agents function as hole or electron injection/transportation, the layers for hole or electron injection/transportation can be omitted correspondingly. While, the materials for hole injection/transportation also function as those for electron injection/transportation, electron injection/transportation layers can be omitted and *vice versa.*

The organic EL elements of the present invention can be applicable for both mono- and multi-layer organic EL elements. The operation of organic EL elements essentially consists of the steps of injecting electrons and holes from electrodes, transporting the electrons and holes through solids, recombining the electrons and holes to generate singlet- or triplet-excitation, and allowing to emit lights from the excitons. These steps essentially do not differ both in the mono- and multi-layer organic EL elements. However, in the monolayer organic EL elements, the features of the above four steps can be improved only by altering the molecular structure of luminescent agents. While the multi-layer organic EL elements, which can allot the functions required in each step to a plurality of materials and independently optimize the materials, are generally composed into a multi-layer form rather than a monolayer form for ease of attaining the desired functions.

In the case of using the 4-cyanocoumarin derivatives of the present invention in dye lasers, they are purified similarly as in conventional dye-laser-active-substances, and then dissolved in appropriate solvents and, if necessary after adjusted to appropriate pH levels, enclosed in dye cells in laser oscillating apparatuses. The 4-cyanocoumarin derivatives have characteristics of a relatively-wide range of stage gains in a visible region, satisfactory-high light tolerance, and desirable quality-durability even after the use of a relatively-long period of time.

The following examples describe the preferred embodiments according to the present invention:

Example 1

4-Cyanocoumarin derivative

Five grams of 9-formyl-8-hydroxy-1,1,7,7-tetramethyl julolidine, represented by Formula 34, and 1.7 g cyanoacetamide were dissolved by heating in methanol. To the solution was added 0.38 ml piperidine, and the mixture was allowed to react for three hours under heating reflux conditions and cooled to ambient temperature. The precipitated 4.1 g crystals of an amide compound represented by Formula 35 were filtered, admixed with 2.9 g *o*-aminothiophenol and 10 ml N,N-dimethylformamide, and reacted at 110°C for six hours. After cooling to ambient temperature, the reaction mixture was admixed with 30 ml isopropyl ether. The formed 4.1 g crystals were collected and dissolved by heating in 300 ml acetone, followed by filtering the solution and distilling the filtrate to remove a half of the acetone. The condensed solution was cooled, and the newly precipitated crystals were filtered, washed with isopropyl ether, and dried to obtain 3.5 g crystals of a compound represented by Formula 36 with a benzothiazolyl group bound to the 3-position.

Formula 34

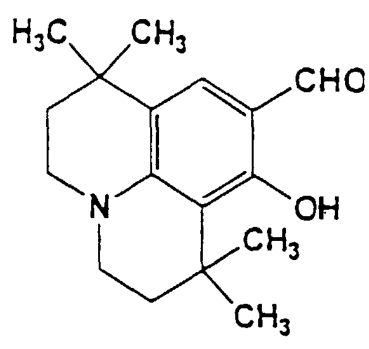

Formula 35

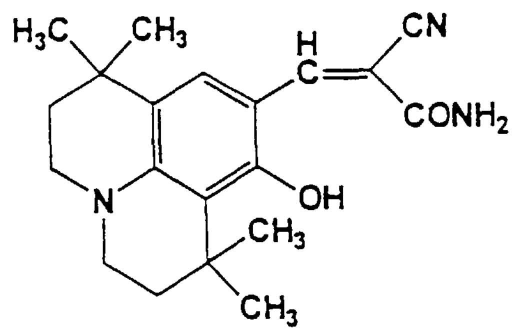

Formula 36

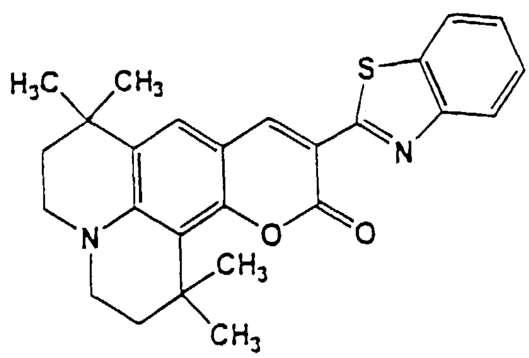

2.3 g of the compound represented by Formula 36 were placed in a reaction vessel, dispersed in 33 ml N,N-dimethylformamide, admixed with 2.53 ml of a 30 w/w % aqueous sodium cyanide drop by drop, reacted for one hour, and further admixed with 0.44 ml bromine drop by drop while cooling to 0-10°C, followed by stirring for two hours. The formed crystals were collected by filtering, washed with water, dried, and purified by silica gel chromatography using chloroform as a developer to obtain 2.1 g glittering green-colored crystals of a 4-cyanocoumarin derivative represented by Formula 3.

Conventional measurement revealed that the 4-cyanocoumarin derivative of this example had a melting point of 266-269°C and showed the absorption maximum and the fluorescent maximum at wavelengths of 564 nm and 614 nm, respectively, when measured in methylene chloride. On measurement of $^1$H-nuclear magnetic resonance spectrum (hereinafter abbreviated as "$^1$H-NMR") in chloroform deuteride, the compound showed a chemical shift δ (ppm, TMS) at peaks of 1.37 (6H, s), 1.59 (6H, s), 1.80 (2H, t), 1.85 (2H, t), 3.36 (2H, t), 3.45 (2H, t), 7.39-7.44 (1H, m), 7.52-7.61 (1H, m), 7.72 (1H, s), 7.96 (1H, d), and 8.18 (1H, d).

Example 2

4-Cyanocoumarin derivative

To 19 g of the amide compound represented by Formula 35, obtained by the method in Example 1, were added 9.2 g of 1-amino-2-thionaphthol and 50 ml N,N-dimethylformamide, followed by a reaction at 110°C for four hours. Thereafter, the reaction mixture was cooled to ambient temperature, and the formed crystals were filtered for collection. The crystals were dissolved in 60 ml chloroform, filtered, and recrystallized in 140 ml methanol. The newly formed crystals were collected by filtering and dried to yield 18.2 g crystals of a compound represented by Formula 37.

Formula 37

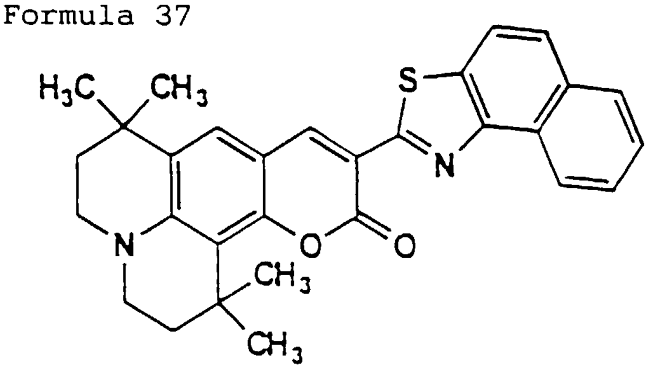

Five grams of the compound represented by Formula 37 were placed in a reaction vessel, dispersed in 50 ml N,N-dimethyl-formamide, admixed with 3.4 ml of a 30 w/w % aqueous sodium cyanide solution drop by drop, and reacted for one hour. To the reaction mixture was added drop by drop 0.59 ml bromine under cooling conditions at 0-10°C, followed by stirring for two hours. 4.9 g of the formed crystals were collected by filtering, washed with water, dried, and recrystallized in N,N-dimethylformamide to obtain 2.6 g glittering green-colored crystals of a 4-cyanocoumarin derivative represented by Formula 10.

Conventional measurement revealed that the 4-cyanocoumarin derivative in this example had a melting point of 322-326°C and showed the absorption maximum and the fluorescent maximum at wavelengths of 580 nm and 627 nm, respectively, when measured in methylene chloride. On measurement of $^1$H-NMR in chloroform deuteride, the compound showed a chemical shift δ (ppm, TMS) at peaks of 1.40 (6H, s), 1.60 (6H, s), 1.81 (2H, t), 1.87 (2H, t), 3.36 (2H, t), 3.45 (2H, t), 7.58-7.63 (1H, m), 7.71-7.76 (1H, m), 7.79 (1H, s), 7.81-7.84 (1H, m), 7.94-7.99 (2H, m), and 9.01 (1H, d).

Example 3

4-Cyanocoumarin derivative

To 3.4 g of the amide compound represented by Formula 35, obtained by the method in Example 1, were added 3.0 g of 2-amino-4-phenylthiophenol and 30 ml N,N-dimethylformamide, followed by a reaction at 140°C for four hours. Thereafter, the reaction mixture was cooled to ambient temperature, and the formed crystals were collected by filtering. The crystals were dissolved in 30 ml chloroform, filtered, and admixed with 70 ml methanol. The newly formed crystals were collected by filtering and dried to yield 2.5 g crystals of a compound represented by Formula 38.

Formula 38

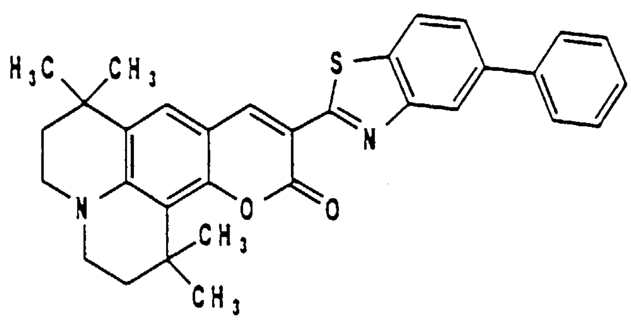

Two and half grams of the compound represented by Formula 38 were placed in a reaction vessel, dispersed in 25 ml N,N-dimethylformamide, admixed drop by drop with 1.6 ml of a 30 w/w % aqueous sodium cyanide solution, and reacted for one hour. To the reaction mixture was added drop by drop 0.31 ml bromine under cooling conditions at 0-10°C, followed by stirring for two hours. The formed crystals were collected by filtering, washed with water, dried, and purified by silica gel column chromatography using chloroform as a developer to obtain 1.9 g glittering green-colored crystals of a 4-cyanocoumarin derivative represented by Formula 12.

Conventional measurement revealed that the 4-cyanocoumarin derivative in this example had a melting point

of 261-263°C and showed the absorption maximum and the fluorescent maximum at wavelengths of 569 nm and 617 nm, respectively, when measured in methylene chloride. On measurement of $^1$H-NMR in chloroform deuteride, the compound showed a chemical shift δ (ppm, TMS) at peaks of 1.38 (6H, s), 1.58 (6H, s), 1.81 (2H, t), 1.85 (2H, t), 3.36 (2H, t), 3.45 (2H, t), 7.26-7.41 (1H, m), 7.46-7.51 (2H, m), 7.66-7.69 (1H, dd), 7.71-7.75 (3H, m), 8.01 (1H, d), and 8.39 (1H, d).

Example 4

4-Cyanocoumarin derivative

To 3.0 g of the amide compound represented by Formula 35, obtained by the method in Example 1, were added 3.0 g of 2-amino-4,5-dimethoxythiophenol and 15 ml N,N-dimethylformamide, followed by a reaction at 140°C for four hours. Thereafter, the reaction mixture was cooled to ambient temperature, and the formed crystals were collected by filtering. The crystals were dissolved in chloroform and purified by silica gel chromatography using a mixture solution of chloroform and ethyl acetate as a developer to yield 2.5 g crystals of a compound represented by Formula 39.

Formula 39

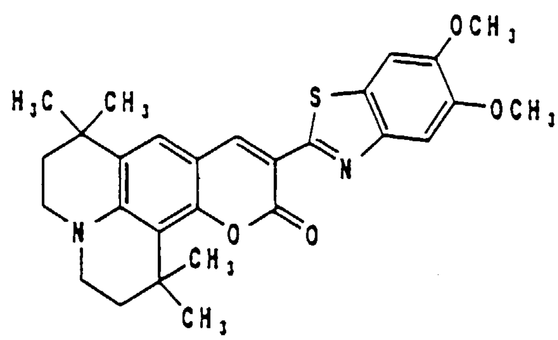

Two and half grams of the compound represented by Formula 39 were placed in a reaction vessel, dispersed in 50 ml N,N-dimethylformamide, admixed with 2.0 ml of a 30 w/w % aqueous sodium cyanide solution drop by drop, and reacted for one hour. To the reaction mixture was added drop by drop 0.25 ml bromine under cooling conditions at 0-10°C, followed by stirring for two hours. The formed crystals were collected by filtering, washed with water, dried, dissolved in chloroform, and purified by silica gel column chromatography using a mixture solution of chloroform and ethyl acetate as a developer to obtain 1.1 g glittering green-colored crystals of a 4-cyanocoumarin derivative represented by the Formula 9.

Conventional measurement revealed that the 4-cyanocoumarin derivative in this example had a melting point of 308-312°C and showed the absorption maximum and the fluorescent maximum at wavelengths of 576 nm and 629 nm, respectively, when measured in methylene chloride. On measurement of $^1$H-NMR in chloroform deuteride, the compound showed a chemical shift δ (ppm, TMS) at peaks of 1.32 (6H, s), 1.60 (6H, s), 1.77 (2H, t), 1.83 (2H, t), 3.29 (2H, t), 3.37 (2H, t), 3.98 (6H, s), 7.36 (1H, s), 7.49 (1H, s), and 7.61 (1H, s).

For the 4-cyanocoumarin derivatives obtained either by the methods in Examples 1 to 4 or in accordance therewith, the properties of the derivatives are tabulated in Table 1. In Table 1, the wavelengths of the absorption maxima of the derivatives were determined after dissolved in methylene chloride, while the fluorescent maxima of the derivatives were determined at a concentration of $10^{-7}$ M in methylene chloride. As controls, compounds represented by Formulae 36 to 39 having the coumarin skeleton were used as related compounds of the above derivatives.

Table 1

| Compound | Absorption maximum (nm) | Fluorescent maximum (nm) | Melting point (°C) | Remarks |
|---|---|---|---|---|
| Formula 3 | 564 | 614 | 266-269 | Present invention |
| Formula 9 | 576 | 629 | 308-312 | Present invention |
| Formula 10 | 580 | 627 | 322-326 | Present invention |
| Formula 11 | 575 | 622 | 302-307 | Present invention |
| Formula 12 | 569 | 617 | 261-263 | Present invention |
| Formula 28 | 586 | 631 | 329-337 | Present invention |
| Formula 36 | 479 | 509 | 224-227 | Control |
| Formula 37 | 489 | 516 | 274-278 | Control |
| Formula 38 | 483 | 511 | 230-234 | Control |
| Formula 39 | 486 | 518 | 263-265 | Control |

As shown in Table 1, the absorption maxima of 4-cyanocoumarin derivatives of the present invention clearly shifted to the side of a longer wavelength as compared with those of the control compounds. All the 4-cyanocoumarin derivatives represented by Formulae 3, 9, 10, 11, 12 and 28 gave absorption maxima nearness to the wavelength of commercially available lighting sources, e.g., a second harmonic of 532 nm of YAG laser. This shows that the 4-cyanocoumarin derivatives of the present invention are useful as photosensitizers for polymerizing polymeric compounds with visible lights. The 4-cyanocoumarin derivatives represented by Formulae 3, 9, 10, 11, 12 and 28 emitted lights in a visible region and showed luminescent maxima, the wavelengths of which were clearly longer than those of the related compounds represented by Formulae 36 to 39. The fact shows that the 4-cyanocoumarin derivatives of the present invention are useful as laser-active substances for dye lasers and as luminescent agents for organic EL elements.

Depending on the structures of the 4-cyanocoumarin derivatives of the present invention, the materials, reaction conditions, and yields thereof are differed, and the derivatives including those represented by Formula 3 to 33 are easily prepared in a desired amount by the methods in Examples 1 to 4 or those in accordance therewith.

Example 5

Photopolymeric composition

According to a usual manner, 900 parts by weight of ethylene glycol monoethyl ether were mixed with 100 parts by weight of pentaerythritol acrylate as a photopolymeric monomer, 100 parts by weight of a copolymer of acrylic acid and methacrylic acid as a binding resin, and eight parts by weight of 3,3',4,4'-tetra(*t*-butylperoxycarbonyl)benzophenone, and further mixed with, as a photosensitizer, six parts by weight of either of the 4-cyanocoumarin derivatives obtained by the methods in Examples 1 and 2. Thus, two types of photopolymeric compositions were obtained.

According to a usual manner, the obtained compositions were homogeneously applied over an aluminum plate, which the surface had been set by sand, to form a photosensitive layer, followed by forming a poly(vinyl alcohol) layer over the photosensitive layer to prevent the polymerization-inhibition by oxygen. A glazing scale was closely attached to the photosensitive layer and a 3 Kw super-high pressure mercury lamp was provided, and the layer was irradiated by a light of a wavelength of 532 nm, which corresponded to a second harmonic of YAG laser, emitted by the lamp using in combination with "Y47" and "Y52", sharp cult filters commercialized by Toshiba Glass Kabushiki-Kaisha, Tokyo, Japan; "KL49" and "KL54", interference filters commercialized by Toshiba Glass Kabushiki-Kaisha, Tokyo, Japan; and "HA30", a heat-ray-cut-filter commercialized by HOYA Corporation, Tokyo, Japan. Thereafter, the irradiated layer was in a usual manner developed in an alkaline developer, followed by calculating the sensitivity based on the step number at which the compositions were photo-cured in such a manner that, in the numerical formula represented by Equation 1, transmittance Tn of the step number "n" of a step tablet, exposure time t, and exposure strength Io were respectively substituted for the equation. In parallel, in place of the 4-cyanocoumarin derivatives of the present invention, there provided systems using related compounds represented by Formulae 36 and 37 and having a similar skeleton as in the derivatives of the present invention represented by Formulae 3 and 10, except that the cyano group at the 4-position of the derivatives was replaced with hydrogen. These related compounds were similarly treated as above for controls. The results are in Table 2.

Equation 1:

$$E(mJ/cm^2) = Io(mJ/cm^2 \cdot s) \times Tn \times t(s)$$

Table 2

| Compound | Wavelength, measured (nm) | Sensitivity ($mJ/cm^2$) | Remarks |
|---|---|---|---|
| Formula 3 | 532 | 0.26 | Present invention |
| Formula 10 | 532 | 0.27 | Present invention |
| Formula 36 | 532 | 1.70 | Control |
| Formula 37 | 532 | 0.32 | Control |

As evident from the results in Table 2, both the 4-cyanocoumarin derivatives represented by Formulae 3 and 10 of the present invention had a significantly higher sensitivity than those of the controls, when measured at a wavelength of 532 nm. This indicates that the derivatives of the present invention can be advantageously used to sensitize

polymeric compounds and polymerization initiators.

Example 6

High-purity 4-cyanocoumarin derivative

The 4-cyanocoumarin derivatives of the present invention, obtained by the methods in Examples 1 to 4, were in a usual manner sublimated for purification into more highly-purified 4-types of 4-cyanocoumarin derivatives represented by Formulae 3, 9, 10 and 12.

The derivatives thus obtained can be extremely useful as luminescent agents in organic EL elements and laser-active substances for dye lasers.

As described above, the present invention was made based on the establishment of novel 4-cyanocoumarin derivatives and the findings of their industrially-useful features. Since the derivatives exert a distinct sensitivity to visible lights, they can be advantageously used as photosensitizers. For example, they can be applied for a variety of fields including the use for photopolymerization where photopolymeric compounds are irradiated, in the presence of or the absence of polymerization initiators, with visible lights such as sun light, carbon arcs, high-pressure mercury lamps, xenon lamps, metal halide lamps, fluorescent lamps, tungsten lamps, argon-ion-lasers, krypton-ion-lasers, helium/cadmium lasers, helium/neon lasers, semiconductor lasers, second harmonic of YAG lasers, etc. Thus, the 4-cyanocoumarin derivatives of the present invention can be advantageously used in the fields, where the photosensitization by visible lights is an essential technical factor, which include lithographic plates for printing such as in laser families, laser printers, color scanners, monotone scanners, photocompositions, planographic processes, gravure printings, flexographies, and silk-screen printings; and another fields of printings, publications, electronics, information recordings, chemicals, metals, automobiles, shipbuildings, and medical industries where inks, paints, chemical vapor depositions (CVD), photosensitive resins, photosensitive films, photosensitive coating agents, photorestoring adhesives, resists for printing substrates, resists for integrated circuits, digital color proofs, holographies, substrates for optical recording disks, fillers for dental therapy, metal letterpress, metal plate finishing, die matrices, shadow masks for color televisions, etc.

Since the 4-cyanocoumarin derivatives of the present invention exert a distinct fluorescent ability in a visible region, they can be quite useful as luminescent agents in organic EL elements and laser-active substances in dye lasers.

The present invention, having these outstanding functions and features, is a significant invention that greatly contributes to this art.

While what are at present considered to be the preferred embodiments of the invention have been described, it will be understood that various modifications may be made therein, and the appended claims are intended to cover all such modifications as fall within the true spirits and scope of the invention.

## Claims

**1.** A 4-cyanocoumarin derivative represented by Formula 1:

Formula 1

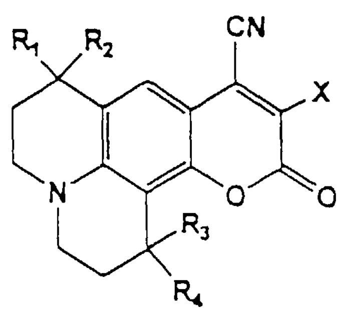

where $R_1$ to $R_4$ independently denote a hydrogen atom, aliphatic hydrocarbon group or aromatic hydrocarbon group, said aliphatic and aromatic hydrocarbon groups may contain a substituent; and X denotes a heterocyclic group which may contain a substituent.

**2.** The derivative of claim 1, wherein $R_1$ to $R_4$ are identical or different and are C 1-4 aliphatic hydrocarbon groups, and X is a polycyclic heterocyclic group including a heterocyclic five-membered ring structure.

**3.** The derivative of claim 1, wherein said aliphatic hydrocarbon group is one or more members selected from the group consisting of a methyl, ethyl, vinyl, propyl, isopropyl, 1-propenyl, 2-propenyl, isopropenyl, butyl, isobutyl, sec-butyl, tert-butyl, 2-butenyl, 1,3-butadienyl, pentyl, isopentyl, neopentyl, and 2-pentenyl groups.

**4.** The derivative of claim 1, wherein said aromatic hydrocarbon group is one or more members selected from the group consisting of a phenyl, tolyl, xylyl, biphenyl, naphthyl, anthryl, and phenanthryl groups.

**5.** The derivative of claim 1, wherein said substituent is one or more members selected from the group consisting of a carboxyl, alkylcarbonyloxy, alkoxy, alkoxycarbonyl, sulfonyl, alkylsulfonyl, aminosulfonyl, hydroxyl, aromatic hydrocarbon, alicyclic hydrocarbon, cyano, 2-butoxyethyl, 2-(2-ethoxy)ethoxyethyl, 2-cyanoethyl, 6-bromohexyl, 2-carboxyethyl, 3-sulfoxypropyl, 4-sulfoxybutyl, 2-hydroxyethyl, phenylmethyl, 4-butoxyphenylmethyl, and 4-butylphenylmethyl groups.

**6.** A 4-cyanocoumarin derivative, which is sensitive to visible light.

**7.** The derivative according to claim 6, which is any one of the 4-cyanocoumarin derivatives of claims 1 to 5.

**8.** A 4-cyanocoumarin derivative, which emits visible light.

**9.** The derivative of claim 8, which is any one of the 4-cyanocoumarin derivatives of claims 1 to 5.

**10.** A photosensitizer which comprises one or more of the 4-cyanocoumarin derivatives of claims 1 to 9.

**11.** A photopolymeric composition, which comprises one or more of the 4-cyanocoumarin derivatives of claims 1 to 9.

**12.** A photopolymeric composition according to claim 11 which can be polymerised by irradiating with visible light.

**13.** A photopolymeric composition according to claim 11 or claim 12 which additionally comprises a polymeric compound and optionally a polymerisation initiator and/or a binding resin.

**14.** The photopolymeric composition of claim 13, wherein said polymeric compound is one or more members selected from the group consisting of ethyl acrylate, hydroxyethyl acrylate, ethylene glycol dimethacrylate, pentaerythritol triacrylate, pentaerythritol trimethacrylate, polyester methacrylate, polyurethane methacrylate, and epoxy methacrylate.

**15.** The photopolymeric composition of claim 13 or claim 14, wherein said polymerization initiator is one or more members selected from the group of consisting of di-*t*-butyldioxyisophtalate, 3,3',4,4'-tetrakis(*t*-butyldioxycarbonyl) benzophenone, ethyl methyl ketone, 2,5-dimethyl-2,5-bis(*t*-butyldioxy)-3-hexane, di-*t*-butylhydroperoxide, 2,5-bis (hydroperoxy)-2,5-dimethylhexane, *t*-butylhydroperoxide, butyl-4,4-bis(*t*-butyldioxy)valylate, 1,1-bis(*t*-butyldioxy)-3,3,5-trimethylcyclohexane, 2,4,6-trichloromethyl-*s*-triazine, bisimidazole, benzoyl alkyl ether, iron-allene complex, titanocene compound, N-phenylglycine, and diphenyliodonium salt.

**16.** The photopolymeric composition of any of claims 13 to 15, wherein said binding resin is one or more members selected from the group consisting of poly-N-vinylpyrrolidone, poly(vinyl acetate), poly(vinyl butyral), poly(vinyl carbazole), polystyrene, poly(methyl methacrylate), poly(ethylene oxide), poly(butyl methacrylate), styrene-maleic esther, poly(methyl methacrylate)methacrylic acid, and poly-N-vinylpyrrolidone-glycidyl methacrylate.

**17.** The photopolymeric composition of any of claims 13 to 16, which comprises to one part by weight of the 4-cyanocoumarin derivative(s) 1-1,000 parts by weight of said polymeric compound(s), and optionally up to 1,000 parts by weight of said binding resin(s).

**18.** A luminescent agent for an organic electroluminescent element, which comprises one or more of the 4-cyanocoumarin derivatives of claims 1 to 9.

**19.** A luminescent agent according to claim 18, which has a luminescent maximum at a wavelength of 600 to 650 nm.

**20.** A laser-active substance, which comprises one or more of the 4-cyanocoumarin derivatives of claims 1 to 9.

**21.** A process for producing a 4-cyanocoumarin derivative of any one of claims 1 to 9, which comprises a step of cyanizing a compound represented by Formula 2 where $R_1$ to $R_4$ and X are as defined in claim 1:

Formula 2

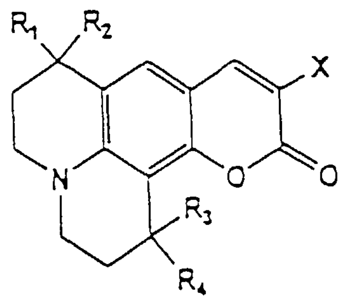

European Patent Office

# EUROPEAN SEARCH REPORT

Application Number
EP 00 30 1950

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 13, no. 1998, 30 November 1998 (1998-11-30) & JP 10 204085 A (MATSUI CHEM. INC.), 4 August 1998 (1998-08-04) * abstract * | 1-3,6-20 | C07D491/16 C09K11/06 |
| X | DE 36 09 804 A (BASF AG) 24 September 1987 (1987-09-24) * claims 1-5; example 9 * | 1-3,6-20 | |
| X | DE 33 22 946 A (BAYER AG) 3 January 1985 (1985-01-03) * claims 1-7; examples 1,2 * | 1-3,6-20 | |
| X | PATENT ABSTRACTS OF JAPAN vol. 2, no. 1996, 29 February 1996 (1996-02-29) & JP 07 267942 A (MITSUI TOATSU CHEM. INC.), 17 October 1995 (1995-10-17) * abstract * | 6,8, 10-13, 18-20 | |
| | | | TECHNICAL FIELDS SEARCHED (Int.Cl.7) |
| X | PATENT ABSTRACTS OF JAPAN vol. 018, no. 215, 18 April 1994 (1994-04-18) & JP 06 009952 A (TDK CORP.), 18 January 1994 (1994-01-18) * abstract * | 6,8, 10-13, 18-20 | C07D C09K |
| X | PATENT ABSTRACTS OF JAPAN vol. 03, no. 1995, 28 April 1995 (1995-04-28) & JP 06 332172 A (TOYOBO CO., LTD.), 2 December 1994 (1994-12-02) * abstract * | 6,8, 10-13, 18-20 | |

-/--

The present search report has been drawn up for all claims

| Place of search | Date of completion of the search | Examiner |
|---|---|---|
| MUNICH | 26 June 2000 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 30 1950

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN vol. 12, no. 1997, 25 December 1997 (1997-12-25) & JP 09 221486 A (MITSUI TOATSU CHEM. INC.), 26 August 1997 (1997-08-26) * abstract * | 6,8, 10-13, 18-20 | |
| X | PATENT ABSTRACTS OF JAPAN vol. 02, no. 1998, 30 January 1998 (1998-01-30) & JP 09 268185 A (MITSUI TOATSU CHEM. INC.), 14 October 1997 (1997-10-14) * abstract * | 6,8, 10-13, 18-20 | |
| X | PATENT ABSTRACTS OF JAPAN vol. 05, no. 1998, 30 April 1998 (1998-04-30) & JP 10 017562 A (MITSUI PETROCHEM. IND., LTD.), 20 January 1998 (1998-01-20) * abstract * | 6,8, 10-13, 18-20 | |
| | | | TECHNICAL FIELDS SEARCHED (Int.Cl.7) |
| X | EP 0 025 136 A (BAYER AG) 18 March 1981 (1981-03-18) * claims 1-8 * | 6,8, 10-13, 18-20 | |
| X | EP 0 101 897 A (BAYER AG) 7 March 1984 (1984-03-07) * claims 1-10 * | 6,8, 10-13, 18-20 | |
| X | DE 32 36 241 A (BAYER AG) 5 April 1984 (1984-04-05) * claims 1-4 * | 6,8, 10-13, 18-20 | |
| | -/-- | | |

The present search report has been drawn up for all claims

| Place of search | Date of completion of the search | Examiner |
|---|---|---|
| MUNICH | 26 June 2000 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 30 1950

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | EP 0 435 262 A (KANSAI PAINT CO., LTD.) 3 July 1991 (1991-07-03)<br>* claims 1-16 *<br>--- | 6,8, 10-13, 18-20 | |
| X | PATENT ABSTRACTS OF JAPAN vol. 016, no. 168, 22 April 1992 (1992-04-22) & JP 04 015201 A (TOYO INK MFG. CO., LTD.), 20 January 1992 (1992-01-20)<br>* abstract *<br>--- | 6,8, 10-13, 18-20 | |
| X | PATENT ABSTRACTS OF JAPAN vol. 04, no. 1997, 30 April 1997 (1997-04-30) & JP 08 337582 A (MITSUI TOATSU CHEM. INC.), 24 December 1996 (1996-12-24)<br>* abstract *<br>--- | 6,8, 10-13, 18-20 | |
| X | PATENT ABSTRACTS OF JAPAN vol. 03, no. 1998, 27 February 1998 (1998-02-27) & JP 09 291087 A (MITSUI TOATSU CHEM. INC.), 11 November 1997 (1997-11-11)<br>* abstract *<br>--- | 6,8, 10-13, 18-20 | TECHNICAL FIELDS SEARCHED (Int.Cl.7) |
| X | PATENT ABSTRACTS OF JAPAN vol. 04, no. 1998, 31 March 1998 (1998-03-31) & JP 09 328482 A (MITSUI PETROCHEM. IND., LTD.), 22 December 1997 (1997-12-22)<br>* abstract *<br>--- | 6,8, 10-13, 18-20 | |
| X | EP 0 797 376 A (BAYER AG) 24 September 1997 (1997-09-24)<br>* claims 1-10 *<br>---<br>-/-- | 6,8, 10-13, 18-20 | |

The present search report has been drawn up for all claims

| Place of search | Date of completion of the search | Examiner |
|---|---|---|
| MUNICH | 26 June 2000 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 30 1950

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | PATENT ABSTRACTS OF JAPAN<br>vol. 01, no. 1998,<br>30 January 1998 (1998-01-30)<br>& JP 09 227547 A (MITSUI TOATSU CHEM. INC.), 2 September 1997 (1997-09-02)<br>* abstract *<br>--- | 6,8,<br>10-13,<br>18-20 | |
| X | PATENT ABSTRACTS OF JAPAN<br>vol. 12, no. 1997,<br>25 December 1997 (1997-12-25)<br>& JP 09 208574 A (MITSUI TOATSU CHEM. INC.), 12 August 1997 (1997-08-12)<br>* abstract *<br>----- | 6,8,<br>10-13,<br>18-20 | |

TECHNICAL FIELDS SEARCHED (Int.Cl.7)

The present search report has been drawn up for all claims

| Place of search | Date of completion of the search | Examiner |
|---|---|---|
| MUNICH | 26 June 2000 | Herz, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 00 30 1950

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2000

| Patent document cited in search report | | Publication date | Patent family member(s) | | Publication date |
|---|---|---|---|---|---|
| JP 10204085 | A | 04-08-1998 | NONE | | |
| DE 3609804 | A | 24-09-1987 | NONE | | |
| DE 3322946 | A | 03-01-1985 | NONE | | |
| JP 07267942 | A | 17-10-1995 | NONE | | |
| JP 06009952 | A | 18-01-1994 | NONE | | |
| JP 06332172 | A | 02-12-1994 | NONE | | |
| JP 09221486 | A | 26-08-1997 | NONE | | |
| JP 09268185 | A | 14-10-1997 | NONE | | |
| JP 10017562 | A | 20-01-1998 | NONE | | |
| EP 25136 | A | 18-03-1981 | DE 2934541 | A | 16-04-1981 |
| | | | DE 2952228 | A | 02-07-1981 |
| | | | DE 3064042 | D | 11-08-1983 |
| | | | JP 1510450 | C | 09-08-1989 |
| | | | JP 56036549 | A | 09-04-1981 |
| | | | JP 63054753 | B | 31-10-1988 |
| | | | US 4544496 | A | 01-10-1985 |
| | | | US 4764622 | A | 16-08-1988 |
| EP 101897 | A | 07-03-1984 | DE 3229301 | A | 12-04-1984 |
| | | | DE 3377060 | D | 21-07-1988 |
| | | | JP 59059683 | A | 05-04-1984 |
| DE 3236241 | A | 05-04-1984 | DE 3372620 | D | 27-08-1987 |
| | | | EP 0105398 | A | 18-04-1984 |
| | | | ES 526117 | D | 16-06-1984 |
| | | | ES 8405833 | A | 01-10-1984 |
| | | | JP 59089302 | A | 23-05-1984 |
| | | | US 4492778 | A | 08-01-1985 |
| EP 435262 | A | 03-07-1991 | CA 2032630 | A,C | 29-06-1991 |
| | | | DE 69026228 | D | 02-05-1996 |
| | | | DE 69026228 | T | 14-11-1996 |
| | | | JP 2930403 | B | 03-08-1999 |
| | | | JP 3223759 | A | 02-10-1991 |
| | | | KR 9401555 | B | 24-02-1994 |
| | | | US 5045434 | A | 03-09-1991 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 00 30 1950

This annex lists the patent family members relating to the patent documents cited in the above-mentioned European search report.
The members are as contained in the European Patent Office EDP file on
The European Patent Office is in no way liable for these particulars which are merely given for the purpose of information.

26-06-2000

| Patent document cited in search report | Publication date | Patent family member(s) | Publication date |
|---|---|---|---|
| JP 04015201 A | 20-01-1992 | NONE | |
| JP 08337582 A | 24-12-1996 | NONE | |
| JP 09291087 A | 11-11-1997 | NONE | |
| JP 09328482 A | 22-12-1997 | NONE | |
| EP 797376 A | 24-09-1997 | DE 19610723 A | 25-09-1997 |
| | | JP 9255947 A | 30-09-1997 |
| JP 09227547 A | 02-09-1997 | NONE | |
| JP 09208574 A | 12-08-1997 | NONE | |

EPO FORM P0459

For more details about this annex : see Official Journal of the European Patent Office, No. 12/82